# EUROPEAN PATENT APPLICATION

(11) **EP 4 374 784 A1**
(43) Date of publication of application: **29.05.2024**
(21) Application number: 23194822.5
(22) Date of filing: 01.09.2023
(51) Int. Cl.: A61B 5/107, A61B 5/00, A61F 2/18, A61B 90/00

(54) **SEPTAL PERFORATION MEASURING DEVICE**

(30) Priority: 23.11.2022 US 202217993440
(71) Applicant: Freudenberg Medical, LLC, Carpinteria, CA 93013 (US)
(72) Inventor: Sowerby, Leigh J., London, N6H 2N7 (CA)
(74) Representative: Schaumburg und Partner Patentanwälte mbB

(57) **Abstract**

A septal perforation measuring device includes an elongated handle and a head portion attached to an end of the elongated handle. The head portion is configured and sized to be inserted into a patient's nostril and includes a base portion and a protrusion extending from a face of the base portion. The protrusion is configured and sized to be inserted into a septal perforation of the patient for assisting in determining a size of the septal perforation.

## Description

### FIELD

The present disclosure relates to a septal perforation measuring device.

### BACKGROUND

This section provides background information related to the present disclosure which is not necessarily prior art.

Nasal septal perforations can be managed with a septal button prosthesis. While they do not restore the physiological function of the septal mucosa, they are able to improve laminar nasal airflow. With the development of septal buttons sized specifically to perforations, accurate measurement of perforations has become more important for patient satisfaction and comfort. This task can be difficult to accomplish in the clinical setting.

The traditional septal buttons were a "one size fits all" type button, so the actual size was not particularly relevant. Newer buttons are now available that are sized specifically to the septal perforation, and as such, accurate measurement of the perforation has become more important for patient satisfaction and comfort. In the clinical setting, the two most common methods currently for measuring perforations are by using a small ruler or simply by "eyeballing" (estimating without a measuring device) the perforation size, but these lead to inaccuracy. Accordingly, it is desirable to provide a septal perforation measuring device that can more accurately measure the septal perforation.

### SUMMARY

This section provides a general summary of the disclosure, and is not a comprehensive disclosure of its full scope or all of its features.

According to an aspect of the present disclosure, a septal perforation measuring device includes an elongated handle and a head portion attached to an end of the elongated handle. The head portion is configured and sized to be inserted into a patient's nostril and includes a base portion and a protrusion extending from a face of the base portion. The protrusion is configured and sized to be inserted into a septal perforation of the patient for assisting in determining a size of the septal perforation.

According to a further aspect, a septal perforation measuring kit includes a plurality of septal perforation measuring devices each having an elongated handle and a head portion attached to an end of the elongated handle. The head portion is configured and sized to be inserted into a patient's nostril and includes a base portion and a protrusion extending from a face of the base portion. The protrusion is configured and sized to be inserted into a septal perforation of the patient. The protrusion of each of the septal perforation measuring devices has one of a different size and shape from the remaining septal perforation measuring devices.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG.1 is a plan view of a septal perforation measuring device according to the principles of the present disclosure:
FIG. 2 is a plan view of a septal perforation measuring device having a different size:
FIG. 3 is a plan view of a septal perforation measuring device having yet another size:
FIG. 4 is a plan view of a septal perforation measuring device according to the principles of the present disclosure having an oval protrusion:
FIG. 5 is a plan view of a septal perforation measuring device with an oval protrusion having a different size:
FIG. 6 is a plan view of a septal perforation measuring device with an oval protrusion having yet another size::
FIG. 7A is a top plan view of a septal perforation measuring device according to the principles of the present disclosure having a stepped protrusion;
FIG. 7B is a side plan view of a septal perforation measuring device shown in FIG. 7A;
FIG. 8A is a top plan view of a septal perforation measuring device according to the principles of the present disclosure having a stepped protrusion with oval shaped steps; and
FIG. 8B is a side plan view of a septal perforation measuring device shown in FIG. 8A.

Corresponding reference numerals indicate corresponding parts throughout the several views of the drawings.

### DETAILED DESCRIPTION

Example embodiments will now be described more fully with reference to the accompanying drawings.

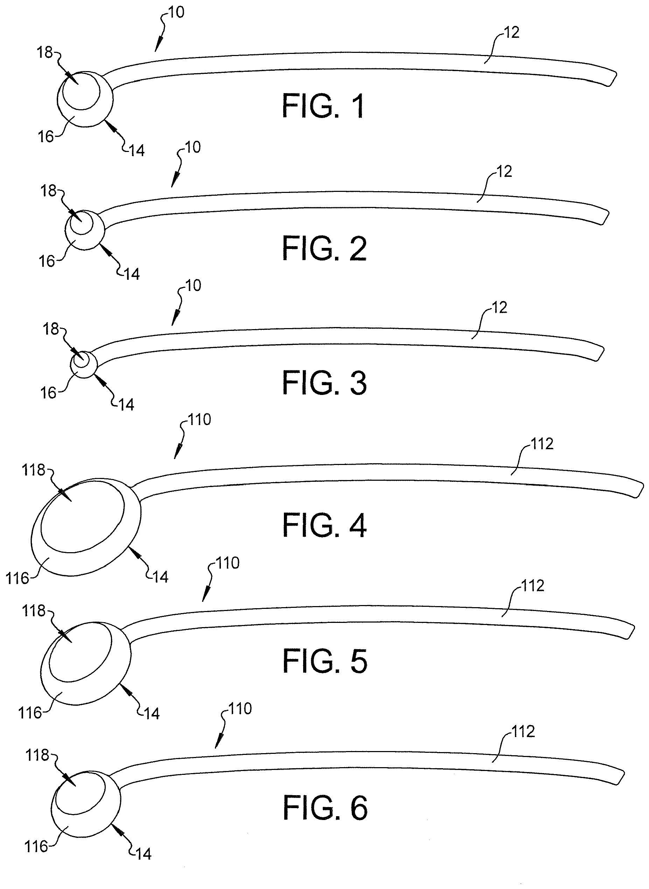

With reference to FIG. 1, a septal perforation measuring device 10 is shown. The septal perforation measuring device 10 includes a handle 12 and a head portion 14 attached to the handle 12. The head portion 14 can include a circular, oblong, or oval base 16 and a protrusion 18 extending from the base 16. The protrusion 18 can be circular, oblong, or oval and is provided with a diameter that is typical of a septal perforation. As shown in FIGS. 1-3, the base 16 of the measuring devices is generally circular and the protrusion 18 is also generally circular. FIGS. 1-3 show the base 16 and the protrusion 18 having differing sizes in the different views. Without intending to be limiting to the specific example sizes, the different sizes as shown, represent a circular 10mm, 7mm and 5mm protrusion diameter, respectively, although other sizes can be used. The sizes can correspond to the different sized septal buttons. The protrusion 18 can be offset relative to a center of the base 16, as shown.

As shown in FIGS. 4-6, the base 116 of the measuring devices 110 is generally oval and the protrusion 118 is also generally oval. FIGS. 4-6 show the base 116 and the protrusion 118 having differing sizes in the different views. Without intending to be limiting to the specific example sizes, the different sizes as shown, represent an oval (length x height) of 21x15 mm, 17x13 mm, and 13x11 mm protrusion dimensions, respectively, although other sizes and shapes can be used. The sizes can correspond to the different sized septal buttons. The protrusion 118 can be offset relative to a center of the base 116, as shown.

The handle 12 as shown is curved, although other shapes, such as straight, can be used. The septal perforation measuring device 10 can be made from metal, such as titanium or steel or from plastic. The different sizing of each device can be printed, engraved, or otherwise labeled on the handle 12 or head portion 14 of the different measuring devices 10.

A kit of numerous septal perforation measuring devices 10 can be provided for allowing an accurate measurement of a patient's septal perforation. In particular, in use, a doctor or technician after observing the septal perforation can take one of the measuring devices by its handle 12 and insert the head portion 14 into the nasal passage and insert the protrusion 18 into the patient's septal perforation. The doctor can evaluate the fit of the protrusion 18 and determine if a larger or smaller or different shaped protrusion 18 would better fit the septal perforation. Once the appropriately sized and shaped protrusion 18 is identified, the doctor or technician can then use that dimension and shape to determine an appropriately sized and shaped septal perforation prosthesis.

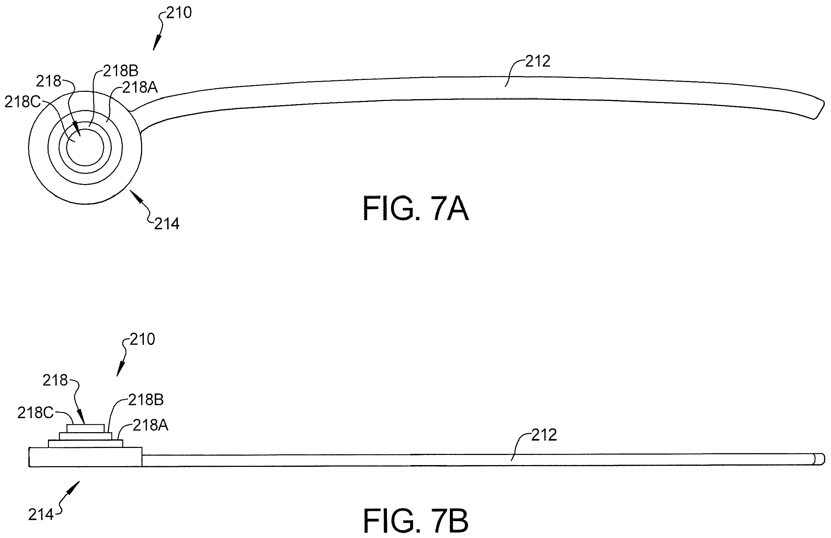

With reference to FIGS. 7A and 7B, an alternative septal perforation measuring device 210 is shown. The septal perforation measuring device 210 includes a handle 212 and a head portion 214 attached to the handle 212. The head portion 214 can include a circular, oblong, or oval base 216 and a protrusion 218 extending from the base 216. The protrusion 218 can include a plurality of different sized circular steps 218A-218C each provided with a diameter that is typical of a septal perforation. Without intending to be limiting to the specific example sizes, the different sizes as shown, represent a circular 10mm, 7mm and 5mm protrusion diameter, respectively, as the steps are located further from the base 216, although other sizes can be used. The sizes can correspond to the different sized septal buttons. The protrusion 218 can be offset relative to a center of the base 216, as shown.

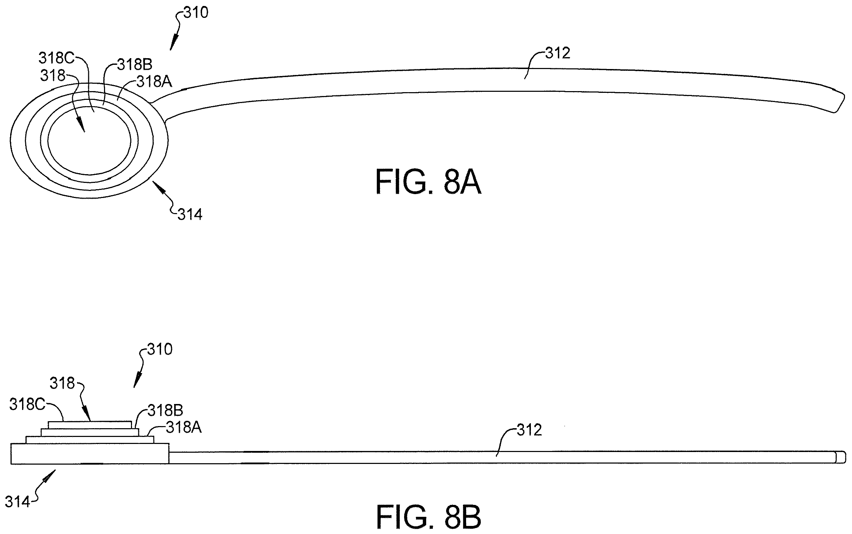

With reference to FIGS. 8A and 8B, a further alternative septal perforation measuring device 310 is shown. The septal perforation measuring device 310 includes a handle 312 and a head portion 314 attached to the handle 312. The head portion 314 can include a circular, oblong, or oval base 816 and a protrusion 318 extending from the base 316. The protrusion 318 can include a plurality of different sized oval steps 318A-318C each provided with dimensions that are typical of a septal perforation. Without intending to be limiting to the specific example sizes, the different sizes as shown, represent an oval (length x height) of 21x15 mm, 17x13 mm, and 13x11 mm protrusion dimensions, respectively, as the steps are located further from the base 316, although other sizes can be used. The sizes can correspond to the different sized oval septal buttons. The protrusion 318 can be offset or centered relative to a center of the base 316, as shown.

In use, a doctor or technician after observing the septal perforation can take one of the measuring devices 210, 310 by its handle 212, 312 and insert the head portion 214, 314 into the nasal passage and insert the protrusion 218, 318 into the patient's septal perforation. The doctor can evaluate which step (218A-218C; 318A-318C) of the protrusion 218, 318 best fits the septal perforation. Once the appropriately sized and shaped protrusion step is identified, the doctor or technician can then use that dimension and shape to determine an appropriately sized and shaped septal perforation prosthesis.

All or some of the above described septal perforation measuring devices can be provided in a kit that allows a doctor or technician to more accurately measure a patient's septal perforation for fitting an appropriately sized septal button or other prosthesis device.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected, or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer, or section. Terms such as "first," "second," and other numerical terms when used herein do not imply a sequence or order unless clearly indicated by the context. Thus, a first element, component, region, layer, or section discussed below could be termed a second element, component, region, layer, or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. A septal perforation measuring device, comprising:
an elongated handle;
a head portion attached to an end of the elongated handle, the head portion being configured and sized to be inserted into a patient's nostril and including a base portion and a protrusion extending from a face of the base portion, the protrusion being configured and sized to be inserted into a septal perforation of the patient.

2. The septal perforation measuring device according to claim 1, wherein the elongated handle is curved.

3. The septal perforation measuring device according to claim 1, wherein the septal perforation measuring device is made from metal.

4. The septal perforation measuring device according to claim 1, wherein the septal perforation measuring device is made from plastic.

5. The septal perforation measuring device according to claim 1, wherein the protrusion includes a plurality of steps each having a decreasing size dimension as each step is further away from the base portion.

6. The septal perforation measuring device according to claim 5, wherein the protrusion is centered relative to the base portion.

7. The septal perforation measuring device according to claim 1, wherein the protrusion is centered relative to the base portion.

8. The septal perforation measuring device according to claim 1, wherein the protrusion is circular.

9. The septal perforation measuring device according to claim 1, wherein the protrusion is oval.

10. A septal perforation measuring kit, comprising:
a plurality of septal perforation measuring devices each having an elongated handle and a head portion attached to an end of the elongated handle, the head portion being configured and sized to be inserted into a patient's nostril and including a base portion and a protrusion extending from a face of the base portion, the protrusion being configured and sized to be inserted into a septal perforation of the patient, the protrusion of each of the septal perforation measuring devices having one of a different size and shape from the remaining septal perforation measuring devices.

11. The septal perforation measuring kit according to claim 10, wherein the elongated handle of each of the plurality of septal perforation measuring devices is curved.

12. The septal perforation measuring kit according to claim 10, wherein each of the septal perforation measuring devices is made from metal.

13. The septal perforation measuring kit according to claim 10, wherein each of the septal perforation measuring devices is made from plastic.

14. The septal perforation measuring kit according to claim 10, wherein the protrusion of each of the plurality of septal perforation measuring devices includes a plurality of steps each having a decreasing size dimension as each step is further away from the base portion.

15. The septal perforation measuring kit according to claim 14, wherein the protrusion is centered on the base portion.

16. The septal perforation measuring kit according to claim 10, wherein the protrusions of the plurality of septal perforation measuring devices are one of circular and oval.
